# EUROPÄISCHE PATENTSCHRIFT

(11) **EP 3 139 835 B1**
(45) Veröffentlichungstag und Bekanntmachung des Hinweises auf die Patenterteilung: **14.10.2020**
(21) Anmeldenummer: 15734555.4
(22) Anmeldetag: 08.05.2015
(51) Int. Cl.: A61B 5/107, A61B 5/103, A61B 5/00

(54) **VERFAHREN ZUR FESTSTELLUNG DER AUSRICHTUNG EINES SYSTEMS UND ANZEIGESYSTEM**
METHOD FOR DETERMINING THE ALIGNMENT OF A SYSTEM, AND A DISPLAY SYSTEM
PROCÉDÉ DE DÉTERMINATION DE L'ORIENTATION D'UN SYSTÈME ET DISPOSITIF D'AFFICHAGE

(30) Priorität: 09.05.2014 DE 102014006690
(43) Veröffentlichungstag der Anmeldung: 15.03.2017
(73) Patentinhaber: Ottobock SE & Co. KGaA, 37115 Duderstadt (DE)
(72) Erfinder: PAPPE, Alexander, 1180 Wien (AU); GLIER, Alexander, 37079 Göttingen (DE); SCHÖNEMEIER, Mark, 37077 Göttingen (DE); PUSCH, Martin, 37115 Duderstadt (DE)
(74) Vertreter: Gramm, Lins & Partner Patent- und Rechtsanwälte PartGmbB
(86) Internationale Anmeldenummer: PCT/DE2015/100186
(87) Internationale Veröffentlichungsnummer: WO 2015/169288

(56) Entgegenhaltungen:
- DE-A1-102012 003 033
- US-A1- 2010 094 174
- US-A1- 2012 271 565

## Beschreibung

Die Erfindung betrifft ein Verfahren zur Feststellung der Ausrichtung oder auch zur Justierung eines Systems aus Mess- und Anzeigeeinrichtung, bei dem mit einer Messeinrichtung, die eine Messplattform aufweist, Horizontal- und Vertikalkräfte sowie ggf. freie Momente erfasst werden, die von einer auf der Messplattform befindlichen Personen ausgeübt werden, bei dem mit zumindest einer Kameraeinrichtung die auf der Messplattform befindliche Person aufgenommen wird, der Anzeigeeinrichtung Bilddaten der zumindest einen Kameraeinrichtung und Messdaten der Messeinrichtung übermittelt werden und eine Kraftwirkungslinie in der Anzeigeeinrichtung den Bilddaten überlagert und auf dem Bild der auf der Messplattform befindlichen Person angezeigt wird. Die Erfindung betrifft ebenfalls ein Anzeigesystem zur Darstellung von Bodenreaktionskräften, die von einer Messplattform erfasst werden, auf der eine Person steht, wobei die Messplattform zwei voneinander entkoppelte Messplatten aufweist, die Sensoren zur Erfassung von Horizontal- und/oder Vertikalkräften und/oder freien Momenten aufweisen, mit zumindest einer Kameraeinrichtung, die die auf der Messplattform befindliche Person zumindest teilweise erfasst und eine Anzeigeeinrichtung, auf der eine Kraftwirkungslinie der Bodenreaktionskräfte der Person überlagert angezeigt werden.

Anzeigesysteme zur Darstellung von Bodenreaktionskräften eines Menschen bei der Einstellung eines Prothesen- oder Orthesenaufbaus ist beispielsweise aus der DE 10 2006 021 788 A1 bekannt. Über die Sensoreinrichtung werden die Vertikal- und Horizontalkomponenten einer Bodenreaktionskraft erfasst und der Kraftvektor der Bodenreaktionskraft wird auf den menschlichen Körper projiziert. Diese Projektion kann gefilmt werden.

Die US 4,598,717 beschreibt ein Anzeigesystem zur Darstellung von Horizontal- und Vertikalkräften, die über Sensoreinheiten aufgenommen wurden. Die Darstellung erfolgt auf einem Monitor, auf dem die Orientierung, der Ort sowie der Betrag der Bodenreaktionskraft angezeigt werden. Über mehrere Kameraeinrichtungen wird das Bild des zu untersuchenden Objektes aufgenommen, die ermittelten Kraftvektoren werden dem Kamerabild überlagert und angezeigt. Das Anzeigesystem ist dazu gedacht, Haltungsschäden sowie Belastungen auf verschiedene Gelenkeinrichtungen während einer Bewegung anzuzeigen.

Die Darstellung von Kraftwirkungslinien auf dem Körper selbst ist schwierig, das Filmen der Person umständlich und die Speicherung und Bearbeitung sowie die Auswertung der Daten, insbesondere die Quantifizierung der Messdaten ist schwierig bis gar nicht darzustellen. Darüber hinaus ist ein Maß an Ungenauigkeit bei der Darstellung vorhanden und eine exakte Ausrichtung und festgelegte geometrische Zuordnung der Projektionseinrichtung zu der Person notwendig.

Die US 2012/271565 A1 betrifft ein trägheitskompensiertes Kraftmesssystem mit einer Kraftmessplatte, die einen Beschleunigungssensor aufweist und zur Übertragung der Messdaten mit einem Computer verbunden ist. Die Messdaten können auch drahtlos übermittelt werden.

Die US 2010/094174 A1 betrifft eine Vorrichtung und ein Verfahren zur Feststellung von muskulo-skeletalen Erkrankungen. Dazu steht eine Person auf einer Kraftmessplatte und wird von mehreren Kameras erfasst. Die Daten der Kraftmessplatte und der Kameras werden einer Anzeigeeinrichtung übermittelt. Für die Untersuchung bringt ein Experte reflektierende oder farbliche Markerpunkte an relevanten Positionen auf der Haut der Person an. Die Markerpunkte werden mit Licht in einem unsichtbaren Spektrum bestrahlt. Die Messdaten und die Bilddaten können mit einer Zeiterkennung gefasst und synchronisiert ausgegeben werden.

Die DE 10/2012 003033 A1 betrifft eine Vorrichtung über ein Verfahren zur Ermittlung und Darstellung von Kräften, bei denen ein resultierender Kraftvektor aus den jeweiligen Unterstützungspunkten zweiter Kraftmessplatten ermittelt wird. Der Kraftvektor wird maßstabsgerecht und Positionsgenau einer auf einem Bildschirm dargestellten Person belagert. Für die Maßstabsgerechte Wiedergabe findet eine Autokallibrierung der Wiedergabeeinrichtung relativ zu den Kraftmessplatten statt, bevor der resultierende Kraftvektor dargestellt wird. Dabei wird ein Lichtfeld ausgehend von der Wiedergabeeinrichtung zunehmend verkleinert, bis jeweils ein an den Kraftmessplatten angeordneter, fotosensitiver Marker Punkt exakt angeleuchtet wird. Die Positionierung der Kraftmessplatten relativ zu der Wiedergabeeinrichtung wird zusammen mit der Brennweite und der Orientierung der Wiedergabeeinrichtung bestimmt.

Aufgabe der vorliegenden Erfindung ist es, ein Verfahren und ein Anzeigesystem bereitzustellen, mit denen insbesondere der Aufbau orthopädischer Einrichtungen wie Orthesen oder Prothesen vermessen, angezeigt und orientiert werden kann, so dass die in die Messplattform eingeleiteten Kräfte korrekt auf einem Bild als Kraftwirkungslinien dargestellt werden können.

Erfindungsgemäß wird die Aufgabe durch ein Verfahren mit den Merkmalen des Hauptanspruches sowie eine Vorrichtung mit den Merkmalen des nebengeordneten Anspruchs gelöst. Vorteilhafte Ausgestaltungen der Erfindung sind in den Unteransprüchen, der Beschreibung sowie den Figuren offenbart.

Das erfindungsgemäße Verfahren zur Feststellung der Ausrichtung eines Systems aus Mess- und Anzeigeeinrichtung mit der Messeinrichtung, die eine Messplattform aufweist, mit der Horizontal- und Vertikalkräfte und/ oder freie Momente erfasst werden, die von einer auf der Messplattform befindlichen Person ausgeübt werden, sieht vor, dass mit zumindest einer Kameraeinrichtung die auf der Messplattform befindliche Person zumindest teilweise aufgenommen wird, der Anzeigeeinrichtung Bilddaten der zumindest einen Kameraeinrichtung und Messdaten der Messeinrichtung übermittelt werden und die Kraftwirkungslinie in der Anzeigeeinrichtung den Bilddaten überlagert und auf dem Bild der auf der Messplattform befindlichen Person angezeigt werden, wobei Markerpunkte mit einem festen geometrischen Bezug zu der Messplattform auf der Messplattform von der zumindest einen Kameraeinrichtung erfasst werden, wobei der geometrische Bezug der Markerpunkte zueinander, insbesondere deren Abstand zueinander, bekannt ist und über eine Bildauswertung die erfassten Markerpunkte die Position und/oder Orientierung der Messplattform zu der zumindest einen Kameraeinrichtung bestimmt wird. Es ist damit möglich, das Mess- und Anzeigesystem zu Justieren, so dass die Kraftwirkungslinie positionsgenau auf dem Bild der Person der Anzeigeeinrichtung dargestellt werden kann, wodurch eine verbesserte Einstellung z.B. eines Prothesenaufbaues erfolgen kann. Durch die Feststellung der Ausrichtung und ggf. Justierung ist es nicht mehr notwendig, eine starre geometrische Zuordnung von Kameraeinrichtung und Messplattform vorzunehmen, vielmehr ist es möglich, über eine frei aufstellbare Kamera eine flexible Aufstellung und einen einfachen Transport des Systems zu ermöglichen. Da kein Projektor mehr der Messplattform zugeordnet werden muss, benötigt das System wesentlich geringere Herstellkosten. Die Bilddaten sowie die Messdaten der Messplattform können einer integrierten Dokumentation zugeführt werden, die sowohl zeitgleich während der Messung als auch nachträglich, beispielsweise im Rahmen einer Nachbesprechung ausgewertet und genutzt werden können. Die Feststellung der Ausrichtung und ggf, die Justierung erfolgt periodisch und/oder nach festgestellten Verschiebungen oder Beschleunigungen der Messplattform oder der Kameraeinrichtung. Um den Rechenaufwand zu verringern, kann vor einer periodischen Feststellung der Ausrichtung und/oder Justierung überprüft werden, ob beispielsweise die Messeinrichtung bewegt wurde, was durch Beschleunigungssensoren in der Messplattform festgestellt werden kann. Die Markerpunkte sind als Glyphen, Leuchtmittel und/oder Reflektoreinrichtungen ausgebildet sein und werden zur Feststellung der Ausrichtung oder Justierung entweder aktiviert oder angestrahlt. Das Aktivieren oder Anstrahlen kann sowohl im sichtbaren als auch im unsichtbaren Lichtspektrum erfolgen.

Eine mobile Ausgestaltung der Anzeigeeinrichtung und eine insgesamt drahtlose Übertragung aller Daten ermöglicht die Involvierung des Patienten in die Messung, damit dieser unmittelbar erkennen kann, welche Auswirkung eine Veränderung eines Aufbaus oder eine Ausrichtung von Orthesen- oder Prothesenkomponenten zueinander auf den Kraftverlauf aufgrund der Darstellung der Kraftwirkungslinie hat. Durch die Speicherungsmöglichkeit der digital vorliegenden Bild- und Messdaten kann eine nachträgliche Auswertung durchgeführt werden, so dass Wiederholungsmessungen vermieden werden können. Darüber hinaus werden Wahrnehmungsfehler auf den Patienten vermieden und eine Unabhängigkeit der Darstellung von den herrschenden Lichtverhältnissen erreicht. Durch die Erfassung der Markerpunkte, die auf der Messplatte angeordnet sind oder zumindest in einer festgelegten und bekannten geometrischen Zuordnung zu der Messplatte stehen, durch die Kameraeinrichtung ist es möglich, bei einem bestimmten und bekannten Abstand der Markerpunkte zueinander über die Bildauswertung die Orientierung, insbesondere die Entfernung der Messplatte zu der Kameraeinrichtung zu bestimmen, so dass es möglich ist, sowohl den genauen Abstand als auch die genaue Orientierung der Kameraeinrichtung zu der Kraftmessplatte und damit auch die exakte Zuordnung der gemessenen Kraftwirkungslinien zu der jeweiligen Position der Kraftmessplatte und damit die zutreffende Position der Kraftwirkungslinie in dem Bild zu errechnen. Dadurch ist es möglich, unabhängig von Lichtverhältnissen, Ausrichteinrichtungen oder dergleichen die jeweiligen Kraftwirkungslinien an dem Ort der Krafteinleitung in die Kraftmessplatte anzuzeigen, so dass eine positionsgenaue Überlagerung der Darstellung der Kraftwirkungslinie mit dem Bild oder Teilbild der auf der Messplattform befindlichen Person gewährleistet ist. Positionsgenau bedeutet, dass die Kraftwirkungslinie auf dem Bild an der Stelle positioniert ist, an der die Bodenreaktionskraft auf die Person einwirkt.

Zur Bestimmung der Orientierung und/oder Position der Kameraeinrichtung relativ zu der Messplattform werden Verzerrungseigenschaften der Kameraeinrichtung berücksichtigt. Jede Kameraeinrichtung weist bestimmte optische oder elektronische Verzerrungseigenschaften auf, die sogenannten intrinsischen Kameraparameter auf, die durch eine Kamerakalibrierung ermittelt werden und zeitlich invariant sind. Sind diese intrinsischen Kameraparameter bekannt, können diese bei der Berechnung der Position der jeweiligen Kraftwirkungslinien relativ zu dem Bild mit berücksichtigt werden, wodurch die Genauigkeit der Darstellung der Kraftwirkungslinie, der Justierung der Anzeige und die Feststellung der Ausrichtung der Komponenten des System zueinander weiter erhöht wird.

Es können periodisch Lichtsignale von dem Leuchtmittel ausgesendet oder zu den Reflektoreinrichtungen ausgesendet werden, um zu überprüfen, ob die Zuordnung der Kameraeinrichtung zu der Messplattform weiterhin gegeben ist und sich nicht verändert hat. Es können bestimmte Leuchtmuster abgefragt werden, um eine Kalibrierung und Neujustierung des Systems durchzuführen. Die Messplattform ist vorteilhafterweise möglichst waagerecht orientiert, was durch eine eingebaute Wasserwaage leicht überprüft werden kann. Über einstellbare Füße kann bei einem unebenen Boden die waagerechte Orientierung gewährleistet werden. Die Kameraeinrichtung ist möglichst orthogonal zu der Messplattform ausgerichtet, wobei bei mehreren Kameraeinrichtungen eine frontal und eine sagittal orientiert ist. Grundsätzlich ist es auch möglich, dass über eine Bilderkennung erfasst wird, ob eine frontale Ausrichtung oder sagittale Ausrichtung vorhanden ist. Es können auch mehrere Kameraeinrichtungen übereinander in der gleichen Blickrichtung orientiert, beispielsweise an einem Träger montiert, angeordnet sein, wodurch die Genauigkeit der Justierung erhöht wird.

Auf der Messplattform sind vorteilhafterweise Markierungen angebracht, um die Ausrichtung des Patienten zu der Messplattform und damit auch zu der Kameraeinrichtung zu standardisieren. Die Füße stehen dabei vorteilhafterweise mit ihrer Längserstreckung entweder senkrecht oder parallel zu der Blickrichtung der Kameraeinrichtung.

Um die Ausrichtung des Systems und ggf. die Justierung weiter zu optimieren, kann über eine Objekterkennung die Messplattform in einem ersten Bildausschnitt erfasst und der Bildausschnitt der Kameraeinrichtung automatisch an einer Markierung der Messplattform oder an einer Seitenkante der Messplattform ausgerichtet werden, so dass beispielsweise die Unterkante des aufgenommenen Bildausschnittes mit der der Kameraeinrichtung zugewandten Vorderkante der Messplattform, die vorzugsweise rechteckig ausgebildet ist, bündig abschließt.

Der Bildausschnitt kann ebenfalls auf der Grundlage einer Objekterkennung der auf der Messplattform befindlichen Personen automatisch eingestellt werden. In der Regel interessiert nicht die Gesamtdarstellung des Patienten, sondern nur die der unteren Extremitäten, so dass hier eine entsprechende Ausschnittseinstellung automatisch erfolgen kann, um die Darstellung auf das Wesentliche zu konzentrieren.

Der Kameraeinrichtung kann eine Leuchteinrichtung zugeordnet sein, von der aus ein Ausrichtstrahl auf die Messplattform projiziert wird, wodurch die Messplattform leichter zu der Kameraeinrichtung ausgerichtet werden kann. Die Ausrichtung kann entweder manuell anhand von auf der Messplattform befindlichen Markierungen erfolgen, alternativ ist zumindest ein Lichtdetektor auf der Messplattform angeordnet, um den Ausrichtstrahl zu erfassen und ein Bestätigungssignal auszulösen, wenn die Kameraeinrichtung korrekt und möglichst optimal zu der Messplattform ausgerichtet ist. Es können auch zwei oder mehr Lichtdetektoren hintereinander angeordnet sein, die so positioniert sind, dass bei einer Detektierung des ausgerichteten Strahles durch alle Lichtdetektoren ein Bestätigungssignal ausgegeben wird. Die Lichtdetektoren können auch als Markierung dienen. Bei einer Aufnahme in der Frontalebene ist es vorteilhaft, wenn die Lichtdetektoren oder -sensoren in der Messplattformmitte in Strahlrichtung hintereinander angeordnet sind, also entlang einer Mittellinie oder zwischen zwei Messplatten, so dass sichergestellt wird, dass die Messplattform im Wesentlichen parallel oder senkrecht zu dem Ausrichtlichtstrahl orientiert und damit auch im Wesentlichen senkrecht oder parallel zu der Blickrichtung der Kameraeinrichtung ausgerichtet ist.

Die Messdaten und die Bilddaten werden bevorzugt drahtlos an die Anzeigeeinrichtung übermittelt, dazu weisen die Komponenten vorteilhafterweise Sende- und ggf. Empfangseinrichtungen auf, so dass die Daten über Netzwerke oder dergleichen übertragen und empfangen werden können. Die Anzeigeeinrichtung ist üblicherweise ein tragbares Anzeigegerät, insbesondere ein Tablet, Computer oder Smartphone, mit denen einerseits eine sehr genaue Anzeige und anderseits eine Speicherung der Messdaten und der Bilddaten vorgenommen werden kann. Das Tablet und dergleichen kann als mobile Anzeigeeinheit sowohl während des Messens von dem Techniker oder von dem Patienten genutzt werden. Darüber hinaus ist es möglich, verschiedene Einstellhilfen in dem Livebild einzublenden, beispielsweise den Abstand der Kraftwirkungslinien zu Referenzlinien oder Markern, die an der Prothese oder der Person befestigt sind.

Die Messdaten und die Bilddaten werden bevorzugt synchron und mit Zeitkennung erfasst und synchronisiert oder kennungsgleich ausgegeben oder gespeichert, so dass es auch grundsätzlich möglich ist, dass das Kamerabild direkt mit Kraftwirkungslinien überlagert angezeigt wird. Kennungsgleich bedeutet, dass die Bilddaten und Messdaten mit der gleichen Zeitkennung einander überlagert werden, um eine realistische Wiedergabe der Kraftwirkungslinie auf dem Bild oder beim Abspielen der Aufnahme während der Messung zu erzielen. Grundsätzlich ist es auch möglich, dass eine statische Aufnahme und eine anschließende Auswertung und Einblendung erfolgt. Die Bilddaten und die Messwerte werden mit einem korrespondierenden Zeitsignal abgespeichert und anschließend ausgewertet, dies kann bei eingeschränkter Online-Rechenleistung vorteilhaft sein. Die Anzeigeeinrichtung kann auf einem Stativ oder einem Träger entweder für die Kamera oder einem separaten Träger abgestellt werden. Das Tablet oder eine andere Anzeigeeinrichtung kann weiterhin mit einem Projektor verbunden oder einem anderen Display verbunden sein, so dass der Inhalt der Anzeigeeinrichtung auch Dritten zugänglich gemacht werden kann.

Werden mehrere Kameraeinrichtungen aus unterschiedlichen Blickwinkeln auf die Messplattform gerichtet, erfolgt vorteilhafterweise eine Feststellung der Ausrichtung und Justierung des Systems und der Anzeige der Kraftwirkungslinie von dem jeweiligen Blickwinkel aus, so dass gleichzeitig die Darstellung auf einem oder mehreren Anzeigeeinrichtungen oder Darstellungen verschiedener Ansichten erfolgen kann. Werden die Bilddaten und die Justier- bzw. Kalibrierdaten mehrerer Kameraeinrichtungen auf die Anzeigeeinrichtung übermittelt, kann dort eine Umschaltung der jeweiligen Ansicht vorgenommen werden, so dass der Techniker oder Patient einen mehrdimensionalen Einblick in den Kraftverlauf erhält.

Zur Verbesserung der Justierung ist es möglich, dass um die Markerpunkte herum Glyphen angeordnet werden, die erfasst und zur Bildauswertung herangezogen werden. Die Markerpunkte selbst können auch als Glyphen ausgebildet sein.

Auf der Person und/oder Messplattform können optische Marker oder Glyphen angebracht sein oder werden, wobei die Kraftwirkungslinie und/oder Referenzlinien entlang der optischen Marker oder Glyphen ausgerichtet sind oder werden. Sowohl die Marker als auch die Glyphen können aktiv oder passiv sein, also entweder selbstständig Licht, auch im nicht sichtbaren Spektrum, ausstrahlen oder von einem Leuchtelement ausgesendete Lichtstrahlen reflektieren. Grundsätzlich ist es auch möglich, dass an dem Tablet oder einer anderen Anzeigeeinrichtung ein Leuchtmittel angebracht ist, um die Reflektoren entsprechend mit Licht zu bestrahlen.

Um bei der Objekterkennung Rechenleistung zu sparen, kann zunächst ein Bild mit einem großen Bildausschnitt aufgenommen werden, wobei die Messplattform über die Objekterkennung erfasst wird und anschließend in den Bereichen der Markerpunkte eine Bilderkennung mit einer höheren oder größeren Auflösung durchgeführt wird. Aufgrund der genaueren Untersuchung der Markerpunkte, die insbesondere an den Ecken der Messplattform angeordnet sind, können die Genauigkeit der Positionserfassung der Marker und damit auch der Orientierung und Positionierung der Kameraeinrichtung relativ zu der Messplattform verbessert erfolgen.

Üblicherweise werden der Aufbau und die Orientierung von Prothesenkomponenten zueinander bei einer gleichmäßigen Belastung beider unteren Extremitäten durchgeführt. Die Messplattform ist vorteilhafterweise zweigeteilt und zumindest kräftemäßig voneinander entkoppelt, so dass einmal das linke Bein und einmal das rechte Bein auf der jeweiligen Kraftmessplatte positioniert wird. Ist eine signifikante Abweichung der Gewichtsverteilung von der rechten Platte zur linken Platte festzustellen, kann ein Warnsignal ausgegeben werden, um zu vermeiden, dass bei einer ungleichmäßigen Belastung ein falscher Aufbau eingestellt wird.

Sämtliche ermittelten Daten der Messplattform können auf dem Tablet oder der Anzeigeeinrichtung angezeigt werden, also die Kraftwirkungslinie, die Kraftgröße, die Verteilung der Kräfte, freie Momente sowie die Änderung der Kräfte.

Neben dem zeitkennungsgleichen Anzeigen von Bildern und Messdaten oder zeitgleich aufgenommener Bild- und Messdaten ist ein entsprechendes Speichern aller vorhandenen Perspektiven und Messdaten vorgesehen. Ebenso können unterschiedliche Ausgabeformate vorgesehen sein, beispielsweise als Bilddaten, als Messdaten oder als PDF-Daten.

Das Anzeigesystem zur Darstellung von Bodenreaktionskräften, die von einer Messplattform erfasst werden, auf der eine Person steht, wobei die Messplattform zwei voneinander entkoppelte Messplatten aufweist, die Sensoren zur Ermittlung von Horizontal- und/oder Vertikalkräften und/oder freien Momenten aufweisen, mit zumindest einer Kameraeinrichtung, die die auf der Messplattform befindlichen Person zumindest teilweise erfasst und eine Anzeigeeinrichtung, auf der die Kraftwirkungslinie der Bodenreaktionskräfte und eine Darstellung der Person überlagert angezeigt werden, sieht vor, dass die Messplattform Markerpunkte mit einem festen geometrischen Bezug zueinander und eine Recheneinheit aufweist, in der vorteilhafterweise die geometrischen Beziehungen, insbesondere Abstände voneinander, abgelegt sind, wobei die Recheneinheit mit der Messplattform, der Kameraeinrichtung und der Anzeigeeinrichtung verbunden oder in einer der Einrichtungen integriert ist. Über die Markerpunkte kann, wie oben ausgeführt, eine automatische Errechnung der Zuordnung über trigonometrische Berechnungen unter Einbeziehung extrinsischer Parameter und intrinsischer Parameter der Kamera erfolgen. Ist die Kamera beispielsweise in einer bestimmten Höhe an einem Stativ befestigt, kann über die Winkelorientierung und die extrinsischen, bekannten Daten der Messplattform und der Markerpositionen zueinander der Abstand und die Orientierung der Kameraeinrichtung relativ zu der Messplattform erfasst werden.

Die Markerpunkte können als Leuchtmittel, Glyphen und/oder Reflektoren ausgebildet sein, insbesondere können die Markerpunkte als ein- und ausschaltbare LED im sichtbaren oder für den Menschen nicht sichtbaren Spektralbereich ausgebildet sein.

Die Messplattform weist neben den Horizontal- und Vertikalkraftsensoren, die in einer ausreichenden Anzahl und Orientierung in oder an der jeweiligen Messplatte angeordnet sind, um die notwendigen Horizontal- und Vertikalkraftkomponenten und freien Momente zu erfassen, einen Beschleunigungssensor auf, der Stöße oder Bewegungen der Messplattform erfasst. Wird durch Betreten oder durch einen Stoß die Messplattform bewegt, wird automatisch eine Neujustierung und Neuerrechnung der relativen Position der jeweiligen Kraftwirkungslinien in dem Bild durchgeführt. Ein solcher Beschleunigungssensor ist auch oder alternativ der Kameraeinrichtung zugeordnet.

Mehrere Kameraeinrichtungen in unterschiedlichen Blickwinkeln können auf die Messplattform gerichtet sein, um ein einfaches Umschalten und eine genauere Darstellung der Kraftwirkungslinien auf dem aufgenommenen Bild zu ermöglichen.

Die Kameraeinrichtung kann an einem Träger angeordnet sein, von dem aus ein Projektor einen Ausrichtstrahl in Richtung auf die Messplattform sendet. Ist zumindest ein Lichtdetektor zur Erfassung des Ausrichtstrahls an der Messplattform angeordnet, kann ein Bestätigungssignal ausgegeben werden, wenn der Ausrichtlichtstrahl den Lichtdetektor oder die Lichtdetektoren trifft. Sofern keine Lichtdetektoren vorgesehen sind, kann über den Ausrichtstrahl eine gewünschte Orientierung leicht durch fluchtendes Ausrichten von Markierungen auf der Messplattform zu dem Ausrichtstrahl erreicht werden.

In der Messplattform oder an der Messplattform kann eine Ausrichteinrichtung angeordnet, insbesondere ausziehbar oder komprimierbar gelagert sein, beispielsweise in Form eines Kunststoffstreifens, der Markierungen aufweist, auf der der Träger der Kameraeinrichtung abzustellen ist. Dadurch wird die gewünschte Ausrichtung erleichtert, ohne dass eine starre geometrische Zuordnung von Kameraeinrichtung und Messplattform erforderlich ist. Insbesondere wird die senkrechte Ausrichtung der Frontkante der Messplattform zu der Blickrichtung der Kamera und gegebenenfalls des Ausrichtstrahls gewährleistet.

Nachfolgend wird ein Ausführungsbeispiel der Erfindung anhand der beigefügten Figuren näher erläutert. Es zeigen:
- Figur 1: eine Gesamtansicht des System;
- Figur 2: eine Darstellung der Anzeigeeinrichtung mit eingeblendetem Kraftwirkungslinie;
- Figur 3: eine Darstellung bei nachträglicher Auswertung und Dokumentation;
- Figur 4: eine schematische Darstellung zur Ermittlung einer relativen räumlichen Position eines Objektes; sowie
- Figur 5: eine Darstellung des Ergebnisses des Justiervorganges.

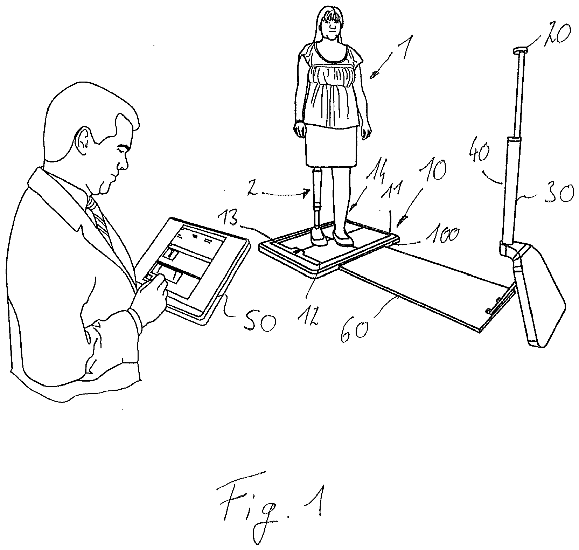

In der Figur 1 ist in einer perspektivischen Darstellung ein System aus einer Messeinrichtung in Gestalt einer Messplattform 10 und einer Kameraeinrichtung 20 gezeigt, die an einem Träger 30 montiert ist. In dem Träger 30, der in seiner Länge zur Einstellung der Höhe der Kameraeinrichtung 20 einstellbar ist, ist eine Leuchteinrichtung 40 angeordnet, um einen Ausrichtlichtstrahl in Richtung auf die Messplattform 10 zu senden. Die Orientierung des Ausrichtlichtstrahls ist vorteilhafterweise identisch mit der zentralen Blickrichtung der Kameraeinrichtung 20. Die Leuchteinrichtung 40 kann beispielsweise als Laserstrahl ausgebildet sein.

Die Messplattform 10 ist in Blickrichtung der Kameraeinrichtung 20 frontal dazu angeordnet, so dass die Vorderkante 100 der im Wesentlichen rechteckigen Messplattform 10 senkrecht zu der Blickrichtung der Kameraeinrichtung 20 orientiert ist. Zur Erleichterung der Ausrichtung von der Kameraeinrichtung 20 zur Messplattform 10 ist an oder in der Messplattform 10 eine Ausrichteinrichtung 60 in Gestalt einer ausziehbaren Matte vorgesehen, an oder in dem der Kameraeinrichtung 20 zugewandten Ende Ausnehmungen oder Markierungen angeordnet sind, die in oder auf die Füße des Trägers 30 gestellt werden, so dass einerseits eine angenähert senkrechte Orientierung der Vorderkante 100 zu der Kameraeinrichtung 20 gewährleistet und andererseits in einer ersten Näherung der Abstand zwischen der Kameraeinrichtung 20 und der Messplattform 10 festgelegt ist. Die Ausrichteeinrichtung 60 kann auch eine Skalierung aufweisen, über die entweder der Abstand der Kameraeinrichtung 20 zu der Vorderkante 100 der Messplattform 10 abgelesen und in eine Recheneinheit eingegeben werden kann, um so eine verbesserte Justierung des Bildes der Kameraeinrichtung zu erreichen. Die Skalierung kann auch von der Kameraeinrichtung 20 mittels einer Bilderkennung erfasst und automatisch eine Recheneinheit übermittelt werden, um auf der Grundlage der erfassten Entfernung die Justierung zumindest vorzubereiten. Grundsätzlich sind jedoch die Kameraeinrichtung 20 mit dem Träger 30 und der Leuchteinrichtung 40 strukturell von der Messplattform 10 entkoppelt, so dass frei wählbare Abstände zwischen der Kameraeinrichtung 20 und der Messplattform 10 vorgesehen werden können; als Einschränkung dient der jeweilige Bildausschnitt der Kameraeinrichtung 20 und die dadurch gegebenenfalls eingeschränkte Erkennbarkeit von Messplattform 10 und der auf der Messplattform 10 befindlichen Person 1 bzw. der Auflösung und der Detektierbarkeit von auf der Messplattform 10 befindlichen Markenpunkten 11, 12, 13, 14.

Vorteilhaft ist, wenn sich die Messplattform 10 und der Träger 30 der Kameraeinrichtung 20 auf einem gleichen Niveau befindet und die Messplattform 10 im Wesentlichen waagerecht ausgerichtet ist. Die Höhe der Kameraeinrichtung 20 über dem Boden kann durch der verstellbaren Träger 30 variiert werden. Dies dient einerseits zum Ausgleich von Höhenunterschieden des Untergrundes und andererseits zur Einstellung des Bildausschnittes, je nach Größe der Person 1 und der zu untersuchenden orthopädietechnischen Einrichtung 2 wie Orthese oder Prothese.

Figur 1 zeigt weiterhin eine Anzeigeeinrichtung 50 in Gestalt eines Tablets, in der auch gleichzeitig eine Recheneinrichtung integriert sein kann, um die Justierung des Mess- und Anzeigesystems durchzuführen. Alternativ zu einer Berechnung in der Anzeigeeinrichtung 50 ist es möglich, dass sowohl die Messdaten der Messplattform 10 als auch die Bilddaten der Kameraeinrichtung 20 an eine separate Recheneinrichtung übermittelt werden, von der dann die aufbereiteten Daten mit dem aufgenommenen Bild der Person 1 und überlagerten Kraftverläufen der Bodenreaktionskräfte an die Anzeigeeinrichtung 50 ebenfalls drahtlos übermittelt werden, so dass dort keine Berechnung selbst erfolgen muss, sondern nur die Anzeige und gegebenenfalls die Speicherung und Weiterverarbeitung durchgeführt wird.

Auf der Messplattform 10, die zwei Messplatten 15, 16 aufweist, die kraft- und momentenmäßig voneinander entkoppelt sind, sind an der Oberseite vier Markerpunkte 11, 12, 13, 14 angeordnet, die entweder als Reflektoren oder als selbst leuchtende Einrichtungen wie LED oder dergleichen ausgebildet sein können. Die Positionen der Markerpunkte 11, 12, 13, 14 zueinander, also auch die Entfernungen der Markerpunkte 11, 12, 13, 14 zueinander sind bekannt und zeitlich unveränderlich, da die Markerpunkte 11, 12, 13, 14 fest an der Messplattform 10 montiert sind. Um die Angriffspunkte der Bodenreaktionskräfte und die Größe sowie Orientierung der Bodenreaktionskräfte auf einem Bild in der Anzeigeeinrichtung 50 korrekt anzeigen oder einblenden zu können, muss die Position auf der Bilddatei richtig eingestellt oder justiert werden. Die Kraftwirkungslinie 70 ist im Prinzip ein dem Bild der Person 1 oder der Prothese 2, wie sie in der Figur 1 dargestellt ist, überlagertes Bild, dessen Positionierung korrekt auf dem Bild der Messplattform 10 zu erfolgen hat. Um diese Position präzise errechnen zu können, wird über die Kameraeinrichtung 20 zunächst die Person 1 und die Messplattform 10 erfasst. Über eine herkömmliche Objekterkennung wird die Messplattform 10 identifiziert und darauf befindliche Markerpunkte 11, 12, 13, 14 ermittelt. Sofern die Markerpunkte 11, 12, 13, 14 aktive Leuchtelemente sind, die sowohl im sichtbaren als auch im unsichtbaren Spektrum arbeiten können, kann von der Recheneinheit oder dem Tablet 50 ein Signal an die Markerpunkte 11, 12, 13, 14 gesendet werden, wenn der Justiervorgang beginnen soll. Bei der der Ausgestaltung der Markerpunkte 11, 12, 13, 14 als Reflektoren werden Lichtsignale, beispielsweise von der Leuchteinrichtung 40, in Richtung auf die Reflektoren gesendet. In der Recheneinheit sind neben den bekannten Entfernungen zwischen den Markerpunkten 11, 12, 13, 14 die Kameraeigenschaften hinsichtlich der Verzerrungseigenschaften in Gestalt von intrinsischen Kameraparametern abgelegt. Die Ermittlung der Kraftwirkungslinienposition auf den Bilddaten erfolgt durch die Errechnung der Beziehungen zwischen den erfassten Markerpunkten 11, 12, 13, 14, den bekannten geometrischen Abmaßen der Messplattform 10 bzw. der geometrischen Beziehungen zwischen den Markerpunkten 11, 12, 13, 14 sowie den intrinsischen Parametern der Kameraeinrichtung 20.

Da keine starre geometrische Zuordnung der Kameraeinrichtung 20 zu der Messplattform 10 vorhanden ist, erfolgt eine regelmäßige Überprüfung der Position der Markerpunkte 11, 12, 13, 14, um eine eventuelle Verschiebung der Messplattform 10 zu der Kameraeinrichtung 20 festzustellen. Ergänzend zu einer rein optischen Auswertung ist es möglich, dass zumindest ein Beschleunigungssensor innerhalb der Messplattform 10 angeordnet ist, über den eine Bewegung der Messplattform 10 erkannt und gegebenenfalls eine Rekalibrierung oder Neujustierung ausgelöst werden kann. Alternativ oder ergänzend kann ein Beschleunigungssensor auch der Kameraeinrichtung 20 zugeordnet sein.

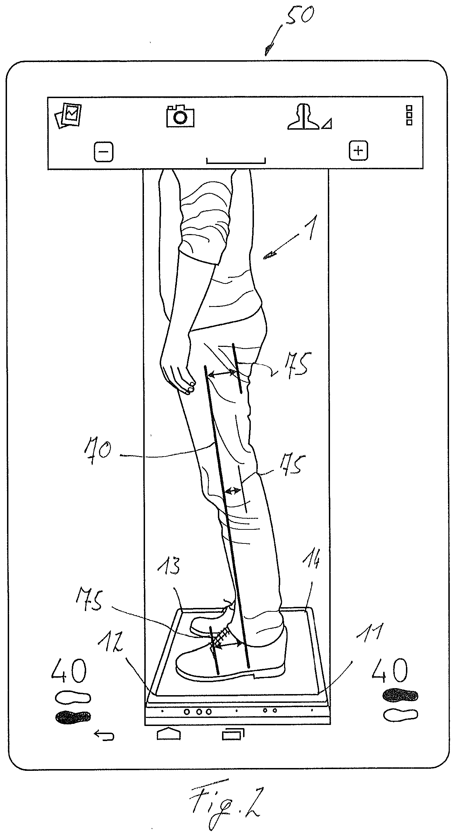

Figur 2 zeigt die Anzeigeeinrichtung 50 in Gestalt eines Tablets. Grundsätzlich sind auch andere Anzeigeeinrichtungen wie Laptops, Computer, Smartphones oder dergleichen geeignet. Auf der Anzeigeeinrichtung 50 ist das Bild der auf der Messplattform 10 stehenden Person 1 zu erkennen. Der Bildausschnitt wurde nach Erkennen der Markerpunkte 11, 12, 13, 14 so gewählt, dass die Unterkante der Messplattform 10 bündig mit dem unteren Bildausschnitt abschließt. Über die Bilderkennung wurde auch der relevante Körperbereich der Person 1 erfasst und eine entsprechende seitliche und höhenmäßige Eingrenzung des Bildausschnittes automatisch vorgenommen. Die Erkennung der Markerpunkte 11, 12, 13, 14 kann verbessert werden, wenn zunächst die Messplattform 10 und die Markerpunkte 11, 12, 13,14 insgesamt erfasst und anschließend mit einer höheren Genauigkeit oder Auflösung der Areale der Markerpunkte 11, 12, 13, 14 überprüft werden. Dies kann durch Glyphen, die um die Markerpunkte 11, 12, 13, 14 herum angeordnet sind, unterstützt werden. Aufgrund der oben beschriebenen Beziehungen zwischen den Markerpunkten 11, 12, 13, 14 und der Bildauswertung der durch die Kameraeinrichtung 20 aufgenommenen Bilder können die durch die Messplattformen 15, 16 für das linke und das rechte Bein getrennt aufgenommenen Bodenreaktionskräfte korrekt in dem Bild eingeblendet werden. Die Kraftwirkungslinie 70 ist dem Bild der Person 1 überlagert, wobei sowohl der Kraftangriffspunkt an der Messplattform 10 oder Messplatte 16 als auch die Orientierung der Bodenreaktionskraft angezeigt wird.

Seitlich neben dem Bildausschnitt ist die Gewichtsverteilung auf beiden Kraftmessplatten 15, 16 dargestellt, im dargestellten Ausführungsbeispiel ist sowohl der rechte als auch der linke Fuß mit jeweils 40kg gleich belastet. Sollte eine zu hohe Differenz zwischen den beiden Gewichtsanzeigen vorliegen, kann über die Anzeigeeinrichtung 50 oder über eine gesonderte Warneinrichtung ein Warnsignal ausgegeben werden, das zur Korrektur der Haltung der Person auffordert, so dass ein Techniker die nicht dargestellte Prothese 50 korrekt einstellen kann.

In der Figur 2 sind ebenfalls Abstände zur Referenzpunkten oder Referenzlinien 75 dargestellt, um dem Techniker die Einstellung eines Prothesenaufbaus zu erleichtern. Im Fußbereich wird der Abstand der Kraftwirkungslinie 70 zu einer vorab festgelegten Referenzposition, die in Abhängigkeit von der Gesamtfußlänge festgelegt ist, angezeigt. Im Kniebereich ist die Referenzlinie der Abstand der Bodenreaktionskraft von dem Kniedrehpunkt, im Hüftbereich der Abstand zum Hüftdrehpunkt.

Sowohl die Messdaten der Messplattform 10 als auch die Bilder können synchronisiert in der Anzeigeeinrichtung 50 gespeichert werden, ebenso können bei mehreren Kameraeinrichtungen 20 sämtliche vorhandenen Perspektiven und Messdaten synchronisiert gespeichert werden, so dass ein nachträgliches Auswerten der Messergebnisse und der Bilddaten leicht erfolgen kann. Sämtliche Datentransportwege erfolgen vorzugsweise kabellos, um eine uneingeschränkte Mobilität zu ermöglichen. Die Referenzlinien 75 oder Referenzpunkte können in dem Bild eingeblendet und verändert werden. Neben der zeitkennungsgleichen Darstellung der Messergebnisse und der aufgenommenen Bilddaten können diese auch als Standbilder ausgegeben und gegebenenfalls ausgedruckt werden. Die jeweilige Perspektive der Kameraeinrichtung 20 kann durch eine Objekterkennung der aufgenommenen Bilddaten automatisch erfolgen.

Neben der Anwendung mehrerer Kameraeinrichtungen aus unterschiedlichen Blickwinkeln, beispielsweise frontal und sagittal, ist es auch möglich, zwei Kameraeinrichtungen übereinander anzuordnen, so dass eine verbesserte Genauigkeit bei der Bildauswertung und der Zuordnung der Bodenreaktionskräfte zu den Bilddaten erfolgen kann.

Die Messplattform 20, ist vorteilhafterweise möglichst senkrecht zu der Blickrichtung der jeweiligen Kameraeinrichtung 20 ausgerichtet, Abweichungen sind jedoch möglich. Die Füße der Person 1 werden zur Erleichterung der Zuordnung in Markierungen auf der Messplattform gestellt oder daran ausgerichtet.

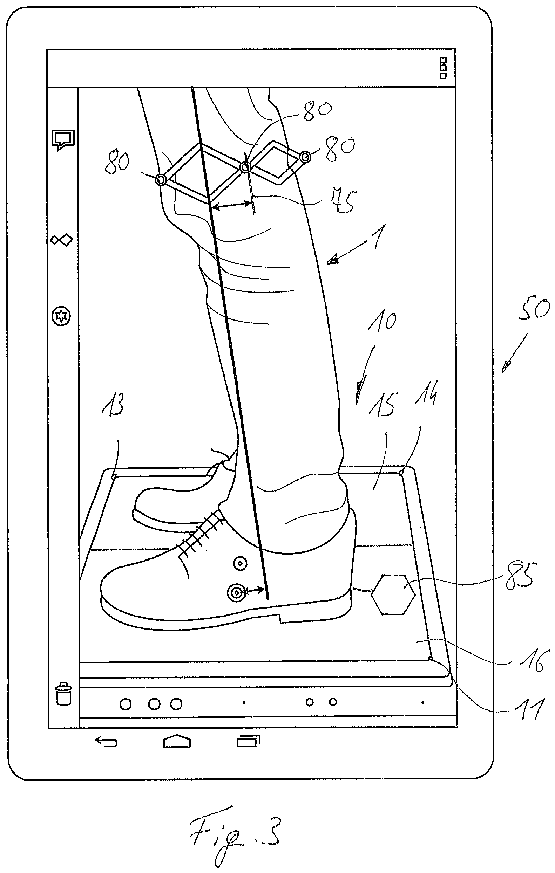

Figur 3 zeigt eine Abbildung einer Messansicht zur nachträglichen Auswertung und Dokumentation, bei der Marker 80 an der Person 1 angeordnet sind. Über die Marker 80, die auch als Glyphen ausgebildet sein können, kann eine erleichterte Zuordnung signifikanter Punkte an einem Prothesenfuß, einem Unterschenkelrohr oder an einem Prothesenkniegelenk vorgenommen werden. Dadurch ist es leichter, den gewünschten Abstand der Kraftwirkungslinie 70 zu den jeweils signifikanten Punkten zu erfassen und die Prothese oder Orthese solange zu verstellen, bis die gewünschten Abstände, die eingezeichnet oder angezeigt werden, erreicht werden. Die Abstände können automatisch über eine Bilderfassung errechnet werden, da aufgrund der bekannten Beziehungen zwischen den Markerpunkten 11, 12, 13, 14 auch eine quantitative Aussage der Lage der Kraftwirkungslinie 70 zu den jeweiligen Markerpunkten 80 getroffen werden kann. Weiterhin ist aufgrund der Speicherung der Bilddaten das Einfügen von Anmerkungen in einem Anmerkungsfeld 85 möglich.

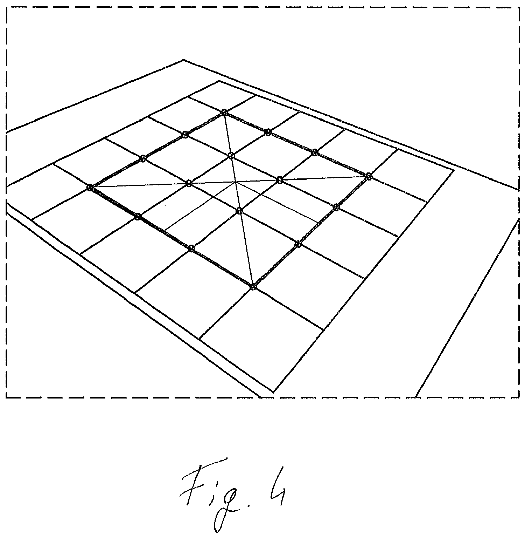

Figur 4 zeigt eine schematische Darstellung einer Kraftmessplatte mit Schachbrettmuster, zur Erläuterung der Errechnung der jeweils zutreffenden Position des Ausgangspunkts der nicht dargestellten Kraftwirkungslinie 70. Über die bekannten Eckpunkte, die in der Figur 4 die Eckpunkte des eingeschlossenen Schachbretts darstellen, ist es möglich, die räumliche Position eines darauf befindlichen Objekts, beispielsweise der Person, und eines fiktiven Objektes, beispielsweise der Kraftwirkungslinie 70, zu errechnen. Neben den intrinsischen Kameraparametern sind die tatsächlichen Abstände der äußeren Eckpunkte des Schachbrettmusters zueinander bekannt, so dass auf der Grundlage trigonometrischer Berechnungen die jeweilige Position errechnet wird.

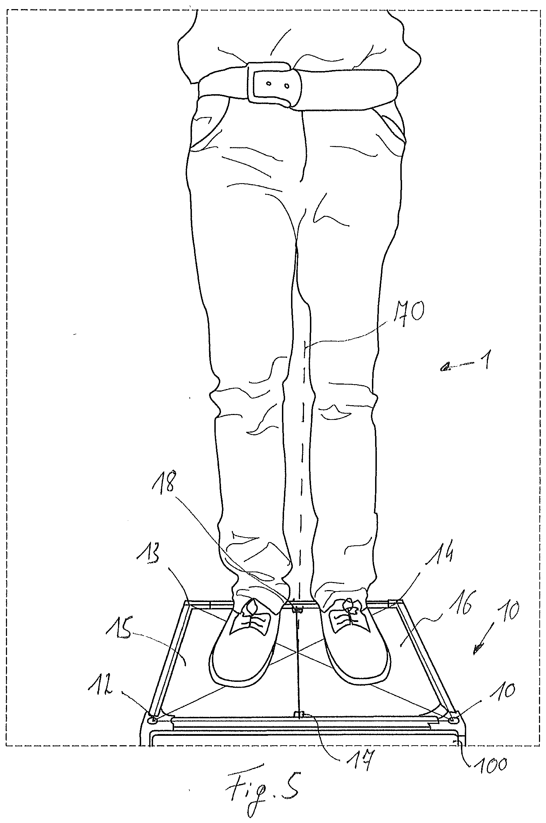

Figur 5 zeigt das Ergebnis eines Justiervorganges, bei der die Vorderkante 100 der Messplattform 10 mit dem unteren Bildausschnitt abschließt. Die vier Markerpunkte 11, 12, 13, 14 sind in den Ecken der Messplattform angeordnet, die Messplattform weist zwei unabhängig voneinander wirksame Messplatten 15, 16 auf, über die Vertikal- und Horizontalkräfte ermittelt werden. Weiterhin sind in der Mitte zwischen den beiden Kraftmessplatten 15, 16 zwei Lichtdetektoren 17, 18 angeordnet, die zur Ausrichtung der Messplattform 10 dienen. Wird ein Ausrichtlichtstrahl in Richtung auf die Messplattform 10 geworfen und verläuft durch beide Lichtdetektoren 17, 18, ist die Messplattform 10 senkrecht dazu ausgerichtet und ein Bestätigungssignal kann ertönen oder ausgegeben werden. Verläuft der Ausrichtlichtstrahl nicht durch beide Lichtdetektoren 17, 18, muss noch eine Korrektur durchgeführt werden.

Die Kraftwirkungslinie 70, hier nur die Schwerpunktlinie, ist dem Bild der Person 1 überlagert, die jeweiligen Koordinaten sind über die Bilderkennung dem Kraftangriffspunkt des Kraftverlaufs 70 zugeordnet.

## Patentansprüche

1. Verfahren zur Feststellung der Ausrichtung eines System aus Mess- und Anzeigeeinrichtungen (10, 50), bei dem
- mit der Messeinrichtung, die eine Messplattform (10) aufweist, Horizontal- und Vertikalkräfte erfasst werden, die von einer auf der Messplattform (10) befindlichen Person (1) ausgeübt werden,
- mit zumindest einer Kameraeinrichtung (20) die auf der Messplattform (10) befindliche Person (1) zumindest teilweise aufgenommen wird,
- der Anzeigeeinrichtung (50) Bilddaten der zumindest einen Kameraeinrichtung (20) und Messdaten der Messeinrichtung übermittelt werden und
- eine Kraftwirkungslinie in der Anzeigeeinrichtung (50) den Bilddaten überlagert und auf dem Bild der auf der Messplattform (10) befindlichen Person (1) angezeigt wird, wobei die Markerpunkte (11, 12, 13, 14) mit einem festen geometrischen Bezug zu der Messplattform (10) von der zumindest einen Kameraeinrichtung (20) erfasst werden, wobei der geometrische Bezug der Markerpunkte (11, 12, 13, 14) zueinander bekannt ist und über eine Bildauswertung der erfassten Markerpunkte (11, 12, 13, 14) die Position und/oder Orientierung der Messplattform (10) zu der zumindest einen Kameraeinrichtung (20) bestimmt wird, wobei eine Justierung periodisch und/oder nach festgestellten Verschiebungen oder Beschleunigungen der Messplattform (10) und/oder der Kameraeinrichtung (20) erfolgt, wobei die Markerpunkte (11, 12, 13, 14) als Glyphen, Leuchtmittel und/oder Reflektoreinrichtungen ausgebildet sind und zur Feststellung der Ausrichtung oder Justierung des Systems aktiviert oder angestrahlt werden.

2. Verfahren nach Anspruch 1, **dadurch gekennzeichnet, dass** zur Bestimmung der Orientierung und/oder Position der Kameraeinrichtung (20) zu der Messplattform (10) Verzerrungseigenschaften der Kameraeinrichtung (20) berücksichtigt werden.

3. Verfahren nach Anspruch 1 oder 2, **dadurch gekennzeichnet, dass** die Leuchtmittel (11, 12, 13, 14) insbesondere in einem sichtbaren Spektrum Licht aussenden und insbesondere die Glyphen um die Markerpunkte (11, 12, 13, 14) herum angeordnet sind, erfasst werden und zur Bildauswertung herangezogen werden.

4. Verfahren nach einem der voranstehenden Ansprüche, **dadurch gekennzeichnet, dass** über eine Objekterkennung die Messplattform (10) erfasst und der Bildausschnitt der Kameraeinrichtung (20) automatisch an einer Markierung der Messplattform (10) oder an einer Seitenkante ausgerichtet wird.

5. Verfahren nach einem der voranstehenden Ansprüche, **dadurch gekennzeichnet, dass** der Bildausschnitt auf der Grundlage eine Objekterkennung der auf der Messplattform (10) befindlichen Person (1) automatisch eingestellt wird.

6. Verfahren nach einem der voranstehenden Ansprüche, **dadurch gekennzeichnet, dass** der Kameraeinrichtung (20) eine Leuchteinrichtung (40) zugeordnet ist, von der aus ein Ausrichtstrahl auf die Messplattform (10) projiziert wird und die Messplattform (10) zu dem Ausrichtstrahl ausgerichtet wird.

7. Verfahren nach einem der voranstehenden Ansprüche, **dadurch gekennzeichnet, dass** mehrere Kameraeinrichtungen (20) aus unterschiedlichen Blickwinkeln auf die Messplattform (10) gerichtet werden und eine Ausrichtung von dem jeweiligen Blickwinkel aus erfolgt.

8. Verfahren nach einem der voranstehenden Ansprüche, **dadurch gekennzeichnet, dass** zunächst ein Bild mit einem großen Bildausschnitt aufgenommen, die Messplattform (10) über eine Objekterkennung erfasst und anschließend in den Bereichen der Markerpunkte (11, 12, 13, 14) eine Objekterkennung mit größere Auflösung durchgeführt wird.

9. Verfahren nach einem der voranstehenden Ansprüche, **dadurch gekennzeichnet, dass** die Kraftwirkungslinie (75) positionsgenau auf dem Bild der Person (1) angezeigt wird.

10. Verfahren nach einem der voranstehenden Ansprüche, **dadurch gekennzeichnet, dass** freie Momente angezeigt werden.

11. Anzeigesystem zur Darstellung von Bodenreaktionskräften, die von einer Messplattform (10) erfasst werden, auf der eine Person (1) steht, die Messplattform (10) weist zwei voneinander entkoppelte Messplatten (15, 16) auf, die Sensoren zur Ermittlung von Horizontal- und/oder Vertikalkräften und/oder freien Momenten aufweisen, mit zumindest einer Kameraeinrichtung (20), die die auf der Messplattform (10) befindliche Person (1) zumindest teilweise erfasst und einer Anzeigeeinrichtung (50), auf der eine Kraftwirkungslinie (70) der Bodenreaktionskräfte und eine Darstellung der Person (1) überlagert angezeigt werden, **dadurch gekennzeichnet, dass** der Messplattform (10) Markerpunkte (11, 12, 13, 14) mit einem festen geometrischen Bezug zueinander zugeordnet sind und das Anzeigesystem eine Recheneinheit aufweist, die mit der Messplattform (10), der Kameraeinrichtung (20) und der Anzeigeeinrichtung (50) verbunden ist, **dadurch gekennzeichnet, dass** die Markerpunkte (11, 12, 13, 14) als Glyphen und/oder Leuchtmittel und/oder Reflektoren ausgebildet sind wobei die Messplattform (10) und/oder die Kameraeinrichtung (20) einen Beschleunigungssensor zur Erkennung von Stößen aufweist und/oder eine Einrichtung zur periodischen Aussendung von Lichtsignalen von dem Leuchtmittel oder zu den Reflektoreinrichtungen vorgesehen ist.

12. Anzeigesystem nach Anspruch 11, **dadurch gekennzeichnet, dass** als Kraftwirkungslinie (70) die Bodenreaktionskräfte dargestellt sind.

13. Anzeigesystem nach Anspruch 11 oder 12, **dadurch gekennzeichnet, dass** mehrere Kameraeinrichtungen (20) in unterschiedlichen Blickwinkeln auf die Messplattform (10) gerichtet sind.

14. Anzeigesystem nach einem der Ansprüche 11 bis 13, **dadurch gekennzeichnet, dass** an der Messplattform (10) eine Ausrichteinrichtung (60) angeordnet ist.

15. Anzeigesystem nach einem der Ansprüche 11 bis 14, **dadurch gekennzeichnet, dass** die Kameraeinrichtung (20) an einem Träger (30) angeordnet ist, von dem aus eine Leuchteinrichtung (40) zumindest ein Ausrichtstrahl in Richtung auf die Messplattform (10) sendet.

16. Anzeigesystem nach einem der Ansprüche 11 bis 15, **dadurch gekennzeichnet, dass** auf der Person (1) und/oder der Messplattform (10) optische Marker (80) und/oder Glyphen angebracht sind und die Kraftwirkungslinie (70) und/oder Referenzlinien (75) entlang der optischen Marker und/oder Glyphen ausgerichtet sind.

17. Anzeigesystem nach einem der Ansprüche 11 bis 16, **dadurch gekennzeichnet, dass** die Kraftwirkungslinie (70) positionsgenau zu dem tatsächlichen Angriffspunkt auf dem Bild der Person (1) dargestellt ist.

## Claims

1. A method for determining the alignment of a system comprising measuring and display devices (10, 50), in which
- using the measuring device, which has a measuring platform (10), horizontal and vertical forces are detected, which are exerted by a person (1) located on the measuring platform (10),
- using at least one camera device (20), the person (1) located on the measuring platform (10) is at least partially photographed,
- image data from the at least one camera device (20) and measurement data from the measuring device are transmitted to the display device (50), and
- a force application line in the display device (50) is superimposed on the image data and is displayed on the image of the person (1) located on the measuring platform (10),
wherein the marker points (11, 12, 13, 14) with a fixed geometric relation to the measuring platform (10) are detected by the at least one camera device (20), wherein the geometric relation of the marker points (11, 12, 13, 14) to one another is known and the position and/or orientation of the measuring platform (10) to the at least one camera device (20) is determined via an image analysis of the detected marker points (11, 12, 13, 14), wherein an adjustment takes place periodically and/or after detected displacements or accelerations of the measuring platform (10) and/or the camera device (20), wherein the marker points (11, 12, 13, 14) are formed as glyphs, illuminants and/or reflector devices and are activated or illuminated to determine the alignment or adjustment of the system.

2. The method according to claim 1, **characterized in that** distortion characteristics of the camera device (20) are considered to determine the orientation and/or position of the camera device (20) relative to the measuring platform (10).

3. The method according to claim 1 or 2, **characterized in that** the illuminants (11, 12, 13, 14) emit light, in particular in a visible spectrum, and in particular the glyphs are arranged around the marker points (11, 12, 13, 14), are detected and are used for image evaluation.

4. The method according to one of the preceding claims, **characterized in that** the measuring platform (10) is detected via an object recognition and the image section of the camera device (20) is aligned automatically on a marking of the measuring platform (10) or on a side edge.

5. The method according to one of the preceding claims, **characterized in that** the image section is set automatically on the basis of an object recognition of the person (1) located on the measuring platform (10).

6. The method according to one of the preceding claims, **characterized in that** a lighting device (40), from which an alignment beam is projected onto the measuring platform (10) and the measuring platform (10) is aligned to the alignment beam, is allocated to the camera device (20)

7. The method according to one of the preceding claims, **characterized in that** a plurality of camera devices (20) are directed to the measuring platform (10) from different viewing angles and an alignment is made from the respective viewing angle.

8. The method according to one of the preceding claims, **characterized in that** an image is initially captured with a large image section, the measuring platform (10) is detected via an object recognition, and an object recognition is subsequently carried out in the areas of the marker points (11, 12, 13, 14) with a higher resolution.

9. The method according to one of the preceding claims, **characterized in that** the force application line (75) is displayed in a positionally accurate manner on the image of the person (1).

10. The method according to one of the preceding claims, **characterized in that** free moments are displayed.

11. A display system for representing ground reaction forces, which are detected by a measuring platform (10), on which a person (1) stands, the measuring platform (10) has two measuring plates (15, 16), which are decoupled from one another and which have sensors for determining horizontal and/or vertical forces and/or free moments, comprising at least one camera device (20), which at least partially detects the person (1) located on the measuring platform (10), and a display device (50), on which a force application line (70) of the ground reaction forces and a representation of the person (1) are displayed in a superimposed manner, **characterized in that** marker points (11, 12, 13, 14) with a fixed geometric relation to one another are allocated to the measuring platform (10), and the display system has processing unit, which is connected to the measuring platform (10), the camera device (20), and the display device (50), **characterized in that** the marker points (11, 12, 13, 14) are formed as glyphs and/or illuminants and/or reflectors, the measuring platform (10) and/or the camera device (20) having an acceleration sensor for detecting shocks and/or a device for periodically emitting light signals from the light source or to the reflector devices being provided.

12. The display system according to claim 11, **characterized in that** the ground reaction forces are represented as force application line (70).

13. The display system according to claim 11 or 12, **characterized in that** a plurality of camera devices (20) are directed to the measuring platform (10) in different viewing angles.

14. The display system according to one of claims 11 to 13, **characterized in that** an alignment device (60) is arranged on the measuring platform (10).

15. The display system according to one of claims 11 to 14, **characterized in that** the camera device (20) is arranged on a support (30), from which a lighting device (40) sends at least one alignment beam in the direction of the measuring platform (10).

16. The display system according to one of claims 11 to 15, **characterized in that** optical markers (80) and/or glyphs are attached to the person (1) and/or the measuring platform (10), and the force application line (70) and/or reference lines (75) are aligned along the optical markers or glyphs.

17. The display system according to one of claims 11 to 16, **characterized in that** the force application line (70) is represented in a positionally accurate manner to the actual contact point on the image of the person (1).

## Revendications

1. Procédé pour fixer l'alignement d'un système constitué d'un dispositif de mesure et d'un dispositif d'affichage (10, 50), dans lequel
- on détecte avec le dispositif de mesure qui comporte une plate-forme de mesure (10) des forces horizontales et verticales exercées par une personne (1) située sur la plate-forme de mesure (10),
- à l'aide d'au moins un dispositif de caméra (20), on enregistre au moins partiellement la personne (1) sur la plate-forme de mesure (10),
- on transmet des données d'image dudit au moins un dispositif de caméra (20) et des données de mesure du dispositif de mesure au dispositif d'affichage (50), et
- on superpose une ligne d'action de force dans le dispositif d'affichage (50) sur les données d'image et on les affiche sur l'image de la personne (1) située sur la plate-forme de mesure (10),
dans lequel
on détecte des points de marquage (11, 12, 13, 14) avec une relation géométrique fixe par rapport à la plate-forme de mesure (10) par ledit au moins un dispositif de caméra (20), la relation géométrique des points de marquage (11, 12, 13, 14) les uns par rapport aux autres étant connue, et on détermine la position et/ou l'orientation de la plate-forme de mesure (10) par rapport audit au moins un dispositif de caméra (20) par une évaluation d'image des points de marquage détectés (11, 12, 13, 14),
on effectue un ajustement périodiquement et/ou après avoir détecté des déplacements ou des accélérations de la plate-forme de mesure (10) et/ou du dispositif de caméra (20),
on réalise les points de marquage (11, 12, 13, 14) sous forme de glyphes, de moyens d'éclairage et/ou de dispositifs réflecteurs et on les active ou on les éclaire pour fixer l'alignement ou l'ajustement du système.

2. Procédé selon la revendication 1,
**caractérisé en ce que** l'on prend en compte des caractéristiques de distorsion du dispositif de caméra (20) pour déterminer l'orientation et/ou la position du dispositif de caméra (20) par rapport à la plate-forme de mesure (10).

3. Procédé selon la revendication 1 ou 2,
**caractérisé en ce que** les moyens d'éclairage (11, 12, 13, 14) émettent de la lumière en particulier dans un spectre visible, et en particulier les glyphes sont disposés autour des points de marquage (11, 12, 13, 14), ils sont détectés et pris en compte pour l'évaluation de l'image.

4. Procédé selon l'une des revendications précédentes,
**caractérisé en ce que** la plate-forme de mesure (10) est détectée au moyen d'une reconnaissance d'objet et la section d'image du dispositif de caméra (20) est automatiquement alignée avec un marquage de la plate-forme de mesure (10) ou avec un bord latéral.

5. Procédé l'une des revendications précédentes,
**caractérisée en ce que** la section d'image est automatiquement ajustée sur la base d'une reconnaissance d'objet de la personne (1) située sur la plate-forme de mesure (10).

6. Procédé selon l'une des revendications précédentes,
**caractérisé en ce qu'**au dispositif de caméra (20) est associé un dispositif d'éclairage (40) à partir duquel un faisceau d'alignement est projeté sur la plate-forme de mesure (10), et la plate-forme de mesure (10) est alignée avec le faisceau d'alignement.

7. Procédé selon l'une des revendications précédentes,
**caractérisée en ce que** plusieurs dispositifs de caméra (20) sont dirigés vers la plate-forme de mesure (10) à partir d'angles de vue différents et un alignement est effectué à partir de l'angle de vue respectif.

8. Procédé selon l'une des revendications précédentes,
**caractérisé en ce que** tout d'abord on enregistre une image avec une grande section d'image, on détecte la plate-forme de mesure (10) au moyen d'une reconnaissance d'objet et ensuite on effectue une reconnaissance d'objet avec une résolution plus élevée dans les zones des points de marquage (11, 12, 13, 14).

9. Procédé selon l'une des revendications précédentes,
**caractérisée en ce que** l'on affiche la ligne d'action de force (75) dans la position exacte sur l'image de la personne (1).

10. Procédé selon l'une des revendications précédentes,
**caractérisé en ce que** l'on affiche des couples libres.

11. Système d'affichage pour représenter les forces de réaction du sol qui sont détectées par une plate-forme de mesure (10) sur laquelle une personne (1) se tient debout, la plate-forme de mesure (10) comportant deux plaques de mesure (15, 16) qui sont découplées l'une de l'autre et qui comportent des capteurs pour déterminer les forces horizontales et/ou verticales et/ou les couples libres, comportant au moins un dispositif de caméra (20) qui détecte au moins partiellement la personne (1) se trouvant sur la plate-forme de mesure (10), et comportant un dispositif d'affichage (50), sur lequel sont superposées une ligne d'action (70) des forces de réaction du sol et une représentation de la personne (1),
**caractérisé en ce que** des points de marquage (11, 12, 13, 14) ayant une relation géométrique fixe les uns par rapport aux autres sont associés à la plate-forme de mesure (10), et le système d'affichage comprend une unité de calcul qui est reliée à la plate-forme de mesure (10), au dispositif de caméra (20) et au dispositif d'affichage (50),
**caractérisé en ce que**
les points de marquage (11, 12, 13, 14) sont réalisés sous forme de glyphes et/ou de moyens d'éclairage et/ou des dispositifs réflecteurs, la plate-forme de mesure (10) et/ou le dispositif de caméra (20) comportant un capteur d'accélération pour la détection d'impacts, et/ou il est prévu un dispositif pour l'émission périodique de signaux lumineux depuis le moyen d'éclairage ou vers les dispositifs réflecteurs.

12. Système d'affichage selon la revendication 11,
**caractérisé en ce que** les forces de réaction du sol sont représentées comme une ligne d'action de force (70).

13. Système d'affichage selon la revendication 11 ou 12,
**caractérisé en ce que**
plusieurs dispositifs de caméra (20) sont dirigés vers la plate-forme de mesure (10) sous différents angles de vue.

14. Système d'affichage selon l'une des revendications 11 à 13, **caractérisé en ce qu'**un dispositif d'alignement (60) est disposé sur la plate-forme de mesure (10).

15. Système d'affichage selon l'une des revendications 11 à 14, **caractérisé en ce que** le dispositif de caméra (20) est disposé sur un support (30), à partir duquel un dispositif d'éclairage (40) émet au moins un faisceau d'alignement en direction de la plate-forme de mesure (10).

16. Système d'affichage selon l'une des revendications 11 à 15, **caractérisé en ce que** des marqueurs optiques (80) et/ou des glyphes sont fixés sur la personne (1) et/ou sur la plate-forme de mesure (10), et la ligne d'action de force (70) et/ou les lignes de référence (75) sont alignées le long des marqueurs optiques et/ou des glyphes.

17. Système d'affichage selon l'une des revendications 11 à 16, **caractérisé en ce que** la ligne d'action de force (70) est représentée sur l'image de la personne (1) dans une position précise par rapport au point d'attaque réel.
